# EUROPEAN PATENT APPLICATION

(11) **EP 4 310 860 A1**
(43) Date of publication of application: **24.01.2024**
(21) Application number: 22771499.5
(22) Date of filing: 17.03.2022
(51) Int. Cl.: G16H 80/00

(54) **COLLECTION DEVICE, COLLECTION METHOD, AND COLLECTION PROGRAM**

(30) Priority: 18.03.2021 JP 2021044693
(71) Applicant: NTT Communications Corporation, Tokyo 100-8019 (JP)
(72) Inventor: SAKURAI, Yoichi, Tokyo 100-8019 (JP); IZUMI, Hiroaki, Tokyo 100-8019 (JP); UMEDA, Takeru, Tokyo 100-8019 (JP); KOKUBO, Ryuta, Tokyo 100-8019 (JP); OTA, Mitsunori, Tokyo 100-8019 (JP)
(74) Representative: Hoffmann Eitle
(86) International application number: PCT/JP2022/012185
(87) International publication number: WO 2022/196754

(57) **Abstract**

A determination unit (15b) determines a date of and an amount of transmission of a medical questionnaire according to a user. A transmitter (15c) transmits a medical questionnaire to the user based on the date of and the amount of transmission. A receiver (15d) receives an answer of the user to the transmitted medical questionnaire and stores the answer in a storage unit (14).

## Description

### [Technical Field]

The present invention relates to a collection device, a collection method, and a collection program.

### [Background Art]

In recent years, in the field of medicine, an ePRO (electric Patient-Reported Outcome) technique by which a patient himself/herself registers daily information, such as pains and quality of sleep, as electric data has been expected. In this technique, a patient answers a questionnaire in a given form, thereby registering electric data in a system. The electric data that is collected in this manner is tallied and analyzed by a third-party organization, such as a medical institute or a pharmaceutical company, with consent of the patient and is utilized for development of new drugs and advanced medicine.

### [Citation List]

### [Non Patent Literature]

"ePRO (patient report outcome system)", [online], January 6th, 2021, Accelight Corporation, [searched on January 6th, 2021], Internet <URL: https://accelight.co.jp/works/product/product-epro/>

### [Summary of Invention]

### [Technical Problem]

Questionnaires however are conventionally in a uniform form that is common to all patients and it is sometimes difficult to collect valid answers. For example, depending on the nature and the life style of patients, valid answers are not necessarily input to all the questionnaires in the uniform form. In some cases, a patient who does not answer a questionnaire until it is close to a deadline date remains forgetting the mere existence of the questionnaire and the date is exceeded or the patient stops the inputting in the middle of a large number of answer items. In some cases, even if a request to answer a questionnaire is made midnight to a patient who is a morning person, the request is sometimes left or remains being forgotten.

The present invention was made in view of the above-described circumstances and an object of the invention is to collect valid answers to a medical questionnaire.

### [Solution to Problem]

In order to solve the above-described problems and achieve the object, a collection device according to the present invention includes: a determination unit configured to determine a date of and an amount of transmission of a medical questionnaire according to a user; a transmitter configured to transmit a medical questionnaire to the user based on the date of and the amount of transmission; and a receiver configured to receive an answer of the user to the transmitted medical questionnaire and store the answer in a storage unit.

### [Advantageous Effects of Invention]

According to the present invention, it is possible to collect valid answers to a medical questionnaire.

### [Brief Description of Drawings]

FIG. 1 is a diagram for describing an overview of a collection device.
FIG. 2 is a schematic view exemplifying a schematic configuration of the collection device.
FIG. 3 is a diagram for describing a process by a determination unit.
FIG. 4 is a flowchart illustrating a procedure of a collection process.
FIG. 5 is a diagram exemplifying a computer that executes a collection program.

### [Embodiments for Carrying Out the Invention]

With reference to the drawings, an embodiment of the present invention will be described in detail below. The embodiment does not limit the invention. In the illustration of the drawings, the same components are denoted with the same reference numerals and are presented.

### [Overview of Collection Device]

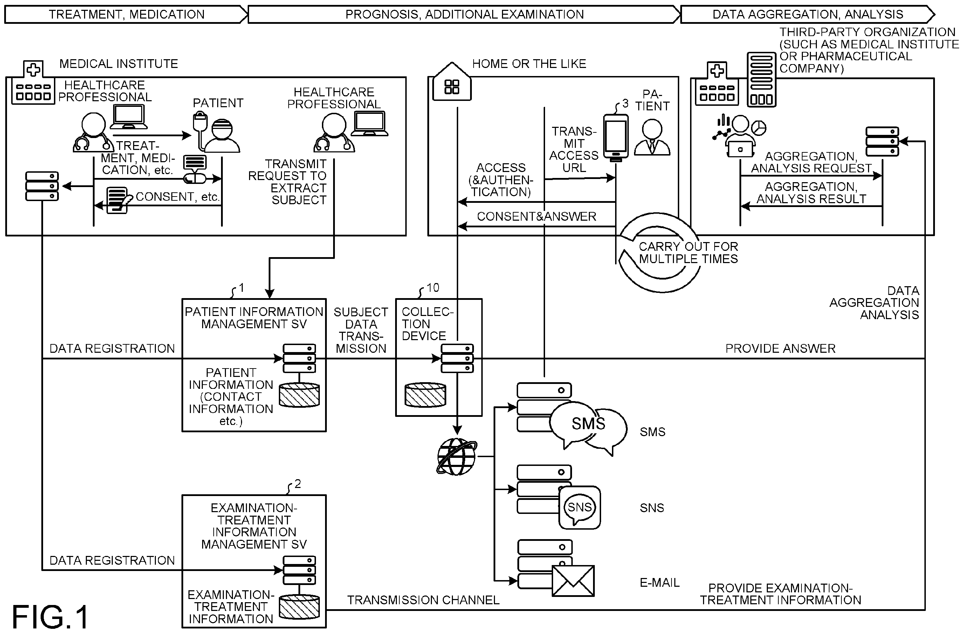

FIG. 1 is a diagram for describing an overview of a collection device. As exemplified in FIG. 1, a system including a collection device 10 is configured by further including a patient information management SV (server) 1, an examination-treatment information management SV 2, and a user terminal device 3 of a user at home.

The patient information management SV 1 manages, patient information containing contact information, etc., in addition to first and last names, a birth date and gender. For example, in a medical institute, initial data of consent data containing a patient number, first and last names, a birth date, gender, and a contact mobile phone number is generated by a patient who receives explanation from a healthcare professional and gives consent for use of examination-treatment information, or the like, for analysis and the initial data is registered as patient information in the patient information management SV 1.

The examination-treatment information management SV 2 manages, in addition to first and last names, a birth date and gender, examination-treatment information containing examination-treatment data, such as content of treatment and a type and a method of medication. For example, in a medical institute, examination-treatment information containing a name, birth date, gender, and examination-treatment data is registered in the examination-treatment information management SV 2 by a healthcare professional.

The collection device 10 collects answers to a medical questionnaire from a patient and manages the answers. For example, the collection device 10 transmits a medical questionnaire for a follow-up of treatment on a patient at home and collects and manages answers of the patient to the medical questionnaire. The collection device 10 acquires patient information from the patient information management SV 1 and, using various contact channels, such as a SMS (Short Message Service), a SNS (Social Networking Service), or E-MAIL, transmits the medical questionnaire to the user terminal device 3 of each patient by a collection process to be described below and receives answers of the patient.

Note that the collection device 10 collects, together with answers, consent information for newly confirming consent for use of answer information and examination-treatment information from a patient and manages the consent information. Thereafter, the answers containing the consent information and the examination-treatment information are associated with each other and are provided to a third-party organization that makes an analysis.

### [Configuration of Collection Device]

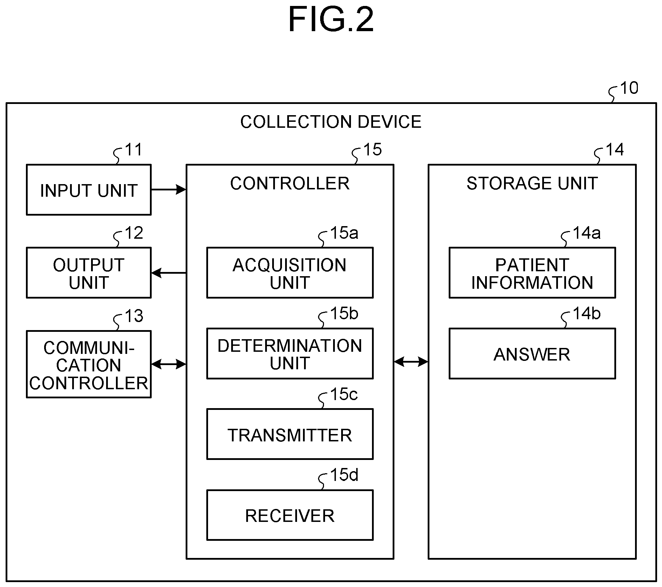

FIG. 2 is a schematic diagram exemplifying a schematic configuration of the collection device. As exemplified in FIG. 2, the collection device 10 is realized by a general-purpose computer, such as a personal computer, and includes an input unit 11, an output unit 12, a communication controller 13, a storage unit 14, and a controller 15.

The input unit 11 is realized using an input device, such as a keyboard and a mouse, and inputs various types of instruction information for start of processing, or the like, to the controller 15 according to an input operation performed by an operator. The output unit 12 is realized by a display device, such as a liquid crystal display, a printing apparatus, such as a printer, or the like.

The communication controller 13 is realized by a NIC (Network Interface Card), or the like, and controls communication between an external device, such as a server, and the controller 15 via a network. For example, the communication controller 13 controls communication between the patient information management SV 1 or the user terminal device 3 of the patient at home and the controller 15.

The storage unit 14 is realized by a semiconductor memory device, such as a RAM (Random Access Memory) or a flash memory, or a storage device, such as a hard disk or an optical disk. In the embodiment, in the storage unit 14, for example, patient information 14a that is used for the collection process to be described below, an answer 14b that is a result of the collection process, etc., are stored. The storage unit 14 may be configured to communicate with the controller 15 via the communication controller 13.

The patient information 14a includes contact information on each patient. The patient information 14a may include, in addition to first and last names, a birth date, gender and contact information, for example, information, such as a requested hours in which each patient answers a medical questionnaire, a log of answers to medical questionnaires in the past, living hours, such as a wake-up time and a time of sleep, and information of a time that can be taken to answer the medical questionnaire. The patient information 14a may include a log of profiling like "A type of patient who is active in trying new drugs" by a healthcare professional on the patient.

The controller 15 is realized using a CPU (Central Processing Unit), or the like, and executes a process program that is stored in a memory. Accordingly, as exemplified in FIG. 2, the controller 15 functions as an acquisition unit 15a, a determination unit 15b, a transmitter 15c and a receiver 15d. Each of or part of these functional units may be implemented in different sets of hardware. For example, the determination unit 15b may be implemented in a device different from the transmitter 15c and the receiver 15d. The controller 15 may include another functional unit.

The acquisition unit 15a acquires the patient information 14a on a user who is a subject of collection. For example, the acquisition unit 15a previously acquires the patient information 14a of the patient on which the collection process to be described below is to be performed via the communication controller 13 from the patient information management SV 1 and stores the patient information 14a in the storage unit 14. Note that the acquisition unit 15a may transmit the acquired patient information 14a immediately to the determination unit 15b at a latter stage without storing the patient information 14a in the storage unit 14.

The determination unit 15b determines a date of transmission of and an amount of a medical questionnaire according to a user. For example, the determination unit 15bc refers to the patient information 14a, determines a date of transmission according to the requested hours and the living hours of the patient, and determines an amount of the questionnaire according to the time that can be taken to answer the medical questionnaire.

Using an advance questionnaire, the determination unit 15b may acquire information of the requested hours, the living hours, the time that can be taken for answering, which are described above, etc., and determine a date of and an amount of transmission according to the answers. For example, the determination unit 15b generates an advance questionnaire, transmits the questionnaire to the user terminal device 3 via the transmitter 15c to be described below, and receives answers via the receiver 15d.

Alternatively, the determination unit 15b determines a date of or an amount of transmission according to a log of behaviors of the user. Specifically, the determination unit 15b refers to the patient information 14a, specifies a tendency of answers using the log of answers of the patient to medical questionnaires in the past, and determines a date of transmission according to the specified tendency. For example, the determination unit 15b determines a date of transmission, putting a limitation to the hours in which the patient answers the medical questionnaires.

According to the tendency of answers of the patient to the medical questionnaires in the past, the determination unit 15b determines an amount of the medical questionnaire to be transmitted at once. For example, when a tendency that a response rate of the patient lowers with respect to a large amount of medical questionnaire in the past can be seen, the determination unit 15b determines an amount to be transmitted at a time such that the amount is under an amount of a medical questionnaire that is not answered or such that the amount does not exceed the amount of the answered medical questionnaire that has been answered

Alternatively, the determination unit 15b determines a date of and an amount of transmission according to a profile of each user that is specified using at least any one of an advance questionnaire, a game and a log of profiling by a healthcare professional.

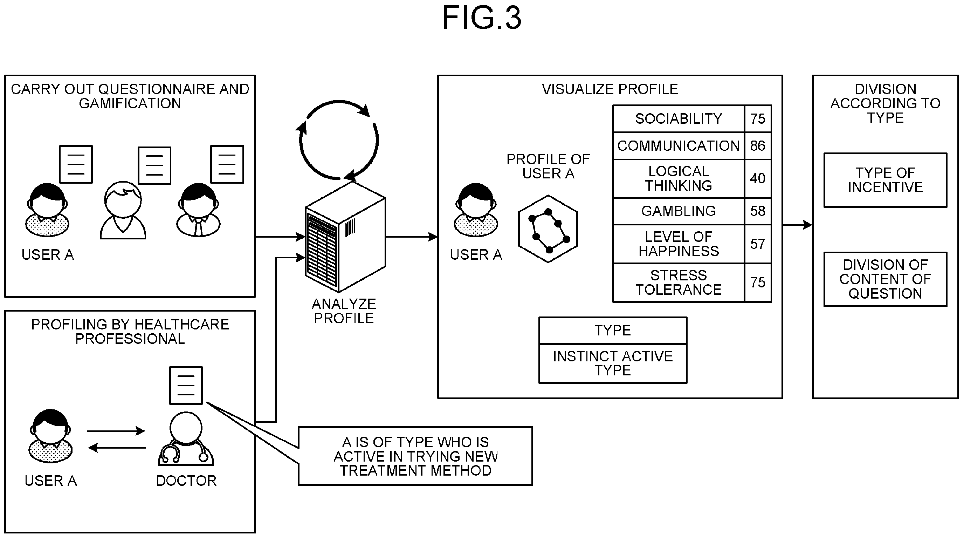

FIG. 3 is a diagram for describing a process by the determination unit. For example, the determination unit 15b asks the patient in advance to execute a given questionnaire, such as a psychological test, or a given game, such as a mental game, to perform patient profiling. Accordingly, for example, as illustrated in FIG. 3, the determination unit 15b performs profiling in view of sociability, communication, logical thinking, gambling, the level of happiness, stress tolerance, etc., and specifies a profile of the patient. In the example illustrated in FIG. 3, User A is specified as an "instinct active type".

The determination unit 15b may specify a profile of the patient using a log of profiling in examination and treatment by a healthcare professional. In the example illustrated in FIG. 3, a log of profiling in examination and treatment by a healthcare professional that is "a type who is active in trying a new treatment method" is registered in the patient information 14a.

The determination unit 15b determines a date of and an amount of transmission of a medical questionnaire according to the specified profile. For example, the determination unit 15b determines a date of and an amount of transmission of a medical questionnaire such that an amount at a time is large or the number of times of transmission is small to a patient of a short-term type. Alternatively, the determination unit 15b determines a date of and an amount of transmission of a medical questionnaire such that an amount at a time is small or the number of times of transmission is large to a patient who is easily bored.

The determination unit 15b may change how to make questions or change how to provide an incentive according to the specified profile. For example, according to the profile of the patient, the determination unit 15b may make a request straightly, make a request humbly, or change the content of questions by presenting choices while taking specific examples. According to the profile of the patient, the determination unit 15b may present a different incentive of providing points as a reward or bringing social contribution for Medicine in Japan or donation to a country in need to the fore.

The transmitter 15c transmits a medical questionnaire to the user based on the date of and the amount of transmission. Specifically, the transmitter 15c divides the medical questionnaire to be transmitted by the number of items corresponding to the amount and transmits the divided medical questionnaire to the user terminal device 3 according to the date of transmission. Accordingly, the transmitter 15c, for example, transmits medical questionnaires of the same content to a patient on which it is determined that the amount at a time is large and the number of times of transmission is small and a patient on which it is determined that, inversely, the amount at a time is small and the number of times of transmission is large in an amount optimum to each patient and at timing optimum to each patient.

The receiver 15d receives the answer 14b of the user to the transmitted medical questionnaire and stores the answer 14b in the storage unit 14. In this manner, the collection device 10 transmits a medical questionnaire in an amount optimum to each patient and at timing optimum to each patient and obtains answers from each patient via the user terminal device 3. This increases the response rate and makes it possible to collect valid answers.

Note that, as described above, the collection device 10 collects, together with answers, consent information for newly confirming consent for use of answer information and examination-treatment information from the patient and manages the consent information. Thereafter, the answers containing the consent information and the examination-treatment information are associated with each other and are provided to a third-party organization that makes an analysis. Thus, the collected valid answers are used for analysis.

### [Collection Process Procedure]

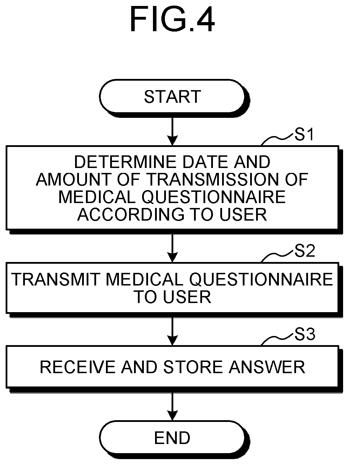

Next, with reference to FIG. 4, the collection process performed by the collection device 10 according to the embodiment will be described. FIG. 4 is a flowchart illustrating a procedure of the collection process. The flowchart in FIG. 4, for example, is started at the timing of an input of an instruction to start the collection process.

First of all, the determination unit 15b determines a date of and an amount of transmission of a medical questionnaire according to a user (step S1). For example, the determination unit 15b refers to the patient information 14a that is on a patient who is a subject of collection and that is acquired by the acquisition unit 15a, determines a date of transmission according to requested hours and living hours of the patient, and determines an amount according to the time that can be taken to answer the medical questionnaire.

Alternatively, the determination unit 15b determines a date or an amount of transmission according to a log of behaviors of the user, such as a tendency of answers of the patient to medical questionnaires in the past.

The determination unit 15b determines a date of and an amount of transmission according to a profile of each user that is specified using at least any one of an advance questionnaire, a game and a log of profiling by a healthcare professional.

The transmitter 15c transmits a medical questionnaire to the user based on the determined date and amount of transmission based (step S2). Specifically, the transmitter 15c divides the medical questionnaire to be transmitted by the number of items corresponding to the amount and transmits the divided medical questionnaire to the user terminal device 3 according to the date of transmission.

The receiver 15d receives the answer 14b of the user to the transmitted medical questionnaire from the user terminal device 3 and stores the answer 14b in the storage unit 14 (step S3). Accordingly, the collection ends.

As described above, in the collection device 10 of the above-described embodiment, the determination unit 15b determines a date of and an amount of transmission of a medical questionnaire according to a user. The transmitter 15c transmits a medical questionnaire to the user based on the date of and the amount of transmission. Specifically, the transmitter 15c divides the medical questionnaire to be transmitted by the number of items corresponding to the amount and transmits the divided medical questionnaire according to the date of transmission. The receiver 15d receives the answer of the user to the transmitted medical questionnaire and stores the answer in the storage unit 14.

As described above, the collection device 10 specifies optimum timing and an optimum amount according to a user of subject to which answers are requested and transmits a questionnaire. This enables the collection device 10 to collect valid answers to the medical questionnaire.

The determination unit 15b determines a date of or an amount of transmission according to the log of behaviors of the user. This makes it possible to specify timing and an amount that are more optimum and collect valid answers.

The determination unit 15b determines a date of and an amount of transmission according to a profile of each user that is specified using at least any one of an advance questionnaire, a game and a log of profiling by a healthcare professional. This makes it possible to specify timing and an amount that are more optimum and collect valid answers.

### [System Configuration, etc.]

Each component of each device illustrated in the drawings is a functional idea and need not necessarily be configured physically as illustrated in the drawings. In other words, specific modes of distribution and integration of devices are not limited to those illustrated in the drawings and all or part of the devices can be configured by functional or physical distribution or integration in any unit according to various types of load and usage. Furthermore, all or given part of each processing function implemented by each device can be realized by a CPU or a GPU (Graphics Processing Unit) and a program that is analyzed and executed by the CPU or the GPU or can be realized as hardware according to a wired logic.

Among the processes described in the above-described embodiment, all or part of the process that is described as one performed automatically can be performed manually or all or part of the process that is described as one performed manually can be performed automatically by a known method. In addition to this, the process procedure, the control procedure, the specific names, and the information including various types of data and parameters that are presented in the description above and the drawings are changeable freely unless otherwise noted.

### [Program]

It is possible to create a program in which the process that collection device described in the above-described embodiment executes is written in a computer-executable language. For example, it is also possible to create a program in which the process executed by the collection device 10 according to the embodiment is written in a computer-executable language. In this case, execution of the program by a computer makes it possible to obtain the same effect as that of the above-described embodiment. Furthermore, the program may be recorded in a computer-readable recording medium and a computer may be caused to read and execute the program that is recorded in the recording medium, thereby realizing the same process as that of the above-described embodiment.

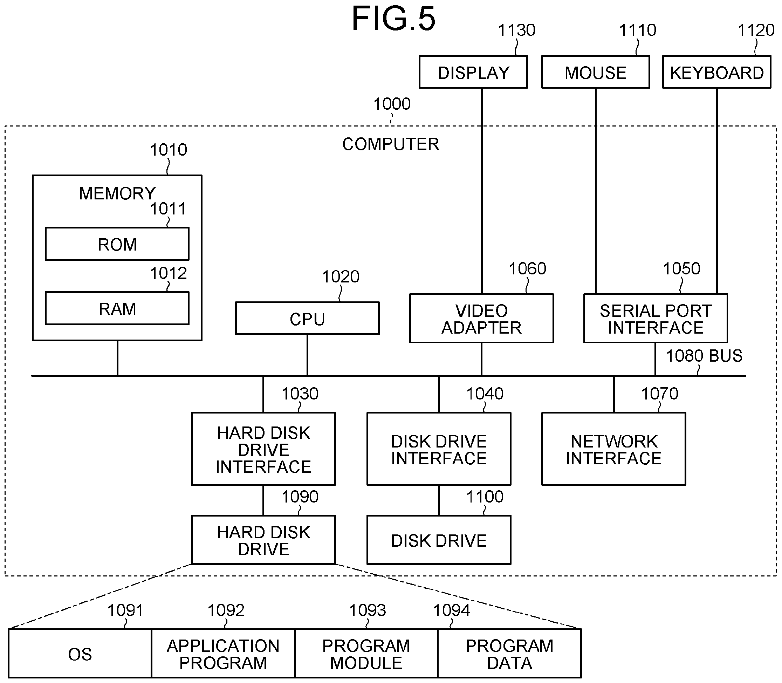

FIG. 5 is a diagram illustrating an example of the computer that executes a collection program. A computer 1000 includes, for example, a memory 1010, a CPU 1020, a hard disk drive interface 1030, a disk drive interface 1040, a serial port interface 1050, a video adapter 1060, and a network interface 1070. Each unit is connected via a bus 1080.

The memory 1010 includes a ROM (Read Only Memory) 1011 and a RAM 1012. The ROM 1011 stores, for example, a boot program, such as a BIOS (Basic Input Output System). The hard disk drive interface 1030 is connected to a hard disk drive 1031. The disk drive interface 1040 is connected to a disk drive 1100. For example, a detachable recording medium, such as a magnetic disk or an optical disk, is inserted into the disk drive 1100. For example, a mouse 1110 and a keyboard 1120 are connected to the serial port interface 1050. For example, a display 1130 is connected to the video adapter 1060.

The hard disk drive 1090 stores, for example, an OS 1091, an application program 1092, a program module 1093, and program data 1094. Each set of information described in the above-described embodiment is stored in, for example, the hard disk drive 1031 or the memory 1010.

The collection program, for example, is stored in the hard disk drive 1090 as the program module 1093 in which commands that are executed by the computer 1000 are written. Specifically, the program module 1093 in which each process executed by the collection device 10 described in the above-described embodiment is written is stored in the hard disk drive 1090.

The data that is used for information processing performed using the provision program is stored as the program data 1094 in, for example, the hard disk drive 1090. The CPU 1020 reads the program module 1093 and the program data 1094 that are stored in the hard disk drive 1090 into the RAM 1012 as requested and executes each of the procedures described above.

The program module 1093 and the program data 1094 according to the collection program are not limited to being stored in the hard disk drive 1090, and the program module 1093 and the program data 1094 may be stored in, for example, a detachable storage medium and may be read by the CPU 1020 via the disk drive 1100, or the like. Alternatively, the program module 1093 and the program data 1094 according to the collection program may be stored in another computer that is connected via a network, such as a LAN (Local Area Network) or a WAN (Wide Area Network), and may be read by the CPU 1020 via the network interface 1070.

The embodiment to which the invention made by the inventors is applied has been described; however, the invention is not limited by the description and the drawings that form part of the disclosure of the invention according to the embodiment. In other words, other embodiments, examples, techniques to be practiced, etc., performed by those skilled in the art based on the embodiment are within the scope of the invention.

### [Explanation of Reference]

- 1: PATIENT INFORMATION MANAGEMENT SV
- 3: USER TERMINAL DEVICE
- 10: COLLECTION DEVICE
- 11: INPUT UNIT
- 12: OUTPUT UNIT
- 13: COMMUNICATION CONTROLLER
- 14: STORAGE UNIT
- 14a: PATIENT INFORMATION
- 14b: ANSWER
- 15: CONTROLLER
- 15a: ACQUISITION UNIT
- 15b: DETERMINATION UNIT
- 15c: TRANSMITTER
- 15d: RECEIVER

## Claims

1. A collection device comprising:
a determination unit configured to determine a date of and an amount of transmission of a medical questionnaire according to a user;
a transmitter configured to transmit a medical questionnaire to the user based on the date of and the amount of transmission; and
a receiver configured to receive an answer of the user to the transmitted medical questionnaire and store the answer in a storage unit.

2. The collection device according to claim 1, wherein the transmitter divides the medical questionnaire to be transmitted by the number of items corresponding to the amount and transmits the divided medical questionnaire according to the date of transmission.

3. The collection device according to claim 1, wherein the determination unit determines the date of or the amount of transmission according to a log of behaviors of the user.

4. The collection device according to claim 1, wherein the determination unit determines the date of and the amount of transmission according to a profile of each user that is specified using at least any one of an advance questionnaire, a game and a log of profiling by a healthcare professional.

5. A collection method executed by a collection device, the collection method comprising:
a determination step of determining a date of and an amount of transmission of a medical questionnaire according to a user;
a transmitting step of transmitting a medical questionnaire to the user based on the date of and the amount of transmission; and
a receiving step of receiving an answer of the user to the transmitted medical questionnaire and stores the answer in a storage unit.

6. A collection program that causes a computer to execute:
determining a date of and an amount of transmission of a medical questionnaire according to a user;
transmitting a medical questionnaire to the user based on the date of and the amount of transmission; and
receiving an answer of the user to the transmitted medical questionnaire and stores the answer in a storage unit.
